(19) Europäisches Patentamt
European Patent Office
Office européen des brevets

(11) Veröffentlichungsnummer: **0 025 191**
**B1**

# (12) EUROPÄISCHE PATENTSCHRIFT

(45) Veröffentlichungstag der Patentschrift: **25.05.83**

(51) Int. Cl.³: **C 07 D 233/54**

(21) Anmeldenummer: **80105114.5**

(22) Anmeldetag: **28.08.80**

(54) **Verfahren zur Herstellung von Imidazolen.**

(30) Priorität: **08.09.79 DE 2936323**

(43) Veröffentlichungstag der Anmeldung: **18.03.81 Patentblatt 81/11**

(45) Bekanntmachung des Hinweises auf die Patenterteilung: **25.05.83 Patentblatt 83/21**

(84) Benannte Vertragsstaaten: **BE CH DE FR GB LI NL**

(56) Entgegenhaltungen:
**DE-A-2 728 976**
**DE-A-2 723 017**
**US-A-3 388 132**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Dockner, Toni, Dr., Grossgasse 6, D-6701 Meckenheim (DE)**
Erfinder: **Kempe, Uwe, Dr., Carl-Bosch-Strasse 44, D-6703 Limburgerhof (DE)**
Erfinder: **Krug, Herbert, Mussbacher Strasse 49, D-6700 Ludwigshafen (DE)**
Erfinder: **Magnussen, Peter, Dr., Professor-Dillinger-Weg 25, D-6702 Bad Duerkheim 1 (DE)**
Erfinder: **Praetorius, Werner, Dr., Dirmsteiner Weg 47, D-6700 Ludwigshafen (DE)**
Erfinder: **Romberg, Helmut, Dr., Amsterdamer Strasse 14, D-6700 Ludwigshafen (DE)**

**EP 0 025 191 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Imidazolen

Die Erfindung betrifft ein neues Verfahren zur Herstellung von Imidazolen durch Umsetzung von N,N'-Diformyl-1,2-diaminen bei Temperaturen von 400 bis 500°C in Gegenwart von Zinkoxid bestimmter Struktur oder einem Gemisch von diesem Zinkoxid und Aluminiumoxid als Katalysator und von Kohlenmonoxid und Wasserstoff in bestimmtem Mengenverhältnis in der Gasphase.

Es ist aus der DE-A-1 952 991 bekannt, daß man Alkylendiamine mit Carbonylverbindungen in Gegenwart eines Kupfer- und/oder Chrom-Katalysators bei 320 bis 650°C zu Imidazolen umsetzen kann. Zusätzlich wird Wasserstoff der Reaktion zugeführt. Die Herstellung von in 2-Stellung unsubstituierten Imidazolen durch Umsetzung der entsprechenden Alkylendiamine mit Ameisensäure oder Formaldehyd gelingt auf diesem Wege jedoch nur in mäßigen Ausbeuten.

Die US-A-2 847 417 beschreibt die Herstellung von Imidazolen aus Alkylendiaminen und Carbonsäuren an Trägerkatalysatoren der Platinmetalle. Solche Katalysatoren sind teuer und werden durch bestimmte Stoffe wie Schwefel, Schwermetalle oder Halogene leicht vergiftet. Edelmetallkatalysatoren verlangen aufwendige Herstellungsverfahren, um die gleichmäßige Wirksamkeit des Katalysators zu gewährleisten, sowie sehr reine Ausgangsstoffe, um die Wirkung des Katalysators nicht zu beeinträchtigen. Zusätzlich wird Wasserstoff zugesetzt, um die Bildung teeriger Polymerer und Ablagerungen auf dem Katalysator zu vermeiden und die Wirksamkeit des Katalysators so möglichst lange zu erhalten.

Nach den Angaben gemäß DE-C-1 231 249, welche der US-A-3 388 132 äquivalent ist, läßt sich Imidazol durch Umsetzung von N,N'-Diformyläthylendiamin in der Gasphase an Zinkoxidkatalysatoren bei Temperaturen zwischen 250 und 800°C herstellen. Die Imidazolausbeute beträgt bei diesem Verfahren zwischen 42 und 80% der Theorie. Alle Beispiele zeigen, daß zusätzlich Stickstoff als Schlepp- oder Verdünnungsmittel zugeführt wird, wobei die Konzentration von 1 bis 999 Mol Stickstoff je Mol Ausgangsstoff beträgt. Nach den Angaben der DE-C-1 231 249 (Spalte 2, Zeilen 29 bis 36), werden die Katalysatoren in einfacher Weise hergestellt und weisen keine besonderen Strukturmerkmale, lediglich eine Korngröße von 0,2 bis 0,4 mm auf. Im Hinblick auf das in der US-PS 2 847 417 beschriebenen Verfahren, dessen Ausbeuten in der Beschreibung als sehr gering angegeben werden, wird kein Wasserstoff verwendet; in allen Beispielen kommt Stickstoff in einer Menge von 45 bis 60 Mol je Mol Ausgangsstoff zur Anwendung.

Die beste Ausbeute gemäß DE-C-1 231 249 an Imidazol von 80% (Beispiel 2) wird mit einem Katalysator von 78 Gewichtsprozent Zinkoxid, 7 Gewichtsprozent Aluminiumoxid, 5 Gewichtsprozent Calciumoxid, 5 Gewichtsprozent Kaliumsulfat, 2 Gewichtsprozent Magnesiumoxid, 1 Gewichtsprozent Chrom(III)-oxid, 2 Gewichtsprozent Eisen(III)-oxid, Natriumoxid und Kaliumoxid bei 500°C in Gegenwart von Stickstoff erhalten.

Die nachgängige DE-A-2 729 017 verwendet im Gegensatz zu dem Verfahren der DE-A-1 231 249 bzw. US-A-3 388 132 keine N,N'-Diformyl-1,2-diamine, sondern setzt 1,2-Diamine mit Ameisensäure, an Zinkoxid bzw. an Zinkoxid und Aluminiumoxid, bevorzugt von bestimmter Struktur, bei 300 bis 600°C um. Es wird ausdrücklich festgestellt, daß bei diesem Katalysator ein Zusatz von Wasserstoff nicht zweckmäßig und auch nicht notwendig ist, da eine Bildung teeriger Polymere, Ablagerungen auf dem Katalysator und ein rascher Rückgang der Katalysatoraktivität nicht beobachtet werden. Seite 7, Absatz 2 und 3 der Offenlegungsschrift zeigen, daß bevorzugt in Korngröße, Porenvolumina und spezifischer Oberfläche strukturierte Katalysatoren verwendet werden. Ein Vergleich der Herstellungsweise von Imidazol (Beispiel 2 der DE-A-2 729 017; Ausbeute 69,2%, bezogen auf umgesetztes Diamin) mit der besten Verfahrensweise der DE-C-1 231 249 bzw. US-A-3 388 132 (Beispiel 2; Ausbeute 80%, bezogen auf angewandtes Diformyläthylendiamin) zeigt, daß der bezüglich Korngröße (0,1 bis 0,3 mm) besonders strukturierte Zinkoxid/Aluminium-Katalysator der DE-A auch in Gegenwart von Stickstoff und einer Temperatur von 550°C eine wesentlich schlechtere Ausbeute als der aus 9 Komponenten und beliebig strukturiertem Zinkoxid gebildete Katalysator im Beispiel 2 der DE-C-1 231 249 bzw. US-A-3 388 132 liefert.

Es wurde nun gefunden, daß man Imidazole der Formel (I)

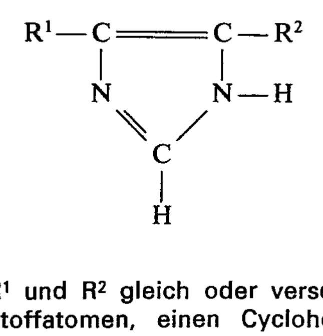

(I)

worin $R^1$ und $R^2$ gleich oder verschieden sein können und jeweils einen Alkylrest mit 1 bis 18 Kohlenstoffatomen, einen Cyclohexylrest, einen Aralkylrest oder Alkylarylrest mit 7 bis 12 Kohlenstoffatomen, einen Phenylrest oder ein Wasserstoffatom bedeuten, durch Umsetzung von

N,N'-Diformyl-1,2-diaminen bei erhöhter Temperatur in Gegenwart von Zinkoxid und gegebenenfalls Aluminiumoxid vorteilhaft erhält, wenn man N,N'-Diformyl-1,2-diamine der Formel (II)

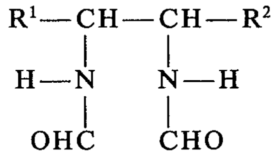

(II)

worin $R^1$ und $R^2$ die vorgenannte Bedeutung besitzen, bei einer Temperatur von 400 bis 500°C in Gegenwart von Zinkoxid mit einem Porenvolumen von 0,05 bis 1 Milliliter je Gramm und einer spezifischen Oberfläche von 1 bis 300 Quadratmeter je Gramm oder einem Gemisch von diesem Zinkoxid und Aluminiumoxid oder dieses Aluminiumoxid enthaltenden Stoffgemischen als Katalysatoren mit oder ohne Träger und in Gegenwart eines Gemischs von Kohlenmonoxid und Wasserstoff mit einem Verhältnis von 1 bis 100 Mol Kohlenmonoxid und 1 bis 100 Mol Wasserstoff je Mol Ausgangsstoff II, gegebenenfalls im Gemisch mit $CO_2$, $NH_3$ und/oder $CH_4$, in der Gasphase umsetzt.

Die Umsetzung kann für den Fall der Verwendung von N,N'-Diformyl-1,2-diaminopropan durch die folgenden Formeln wiedergegeben werden:

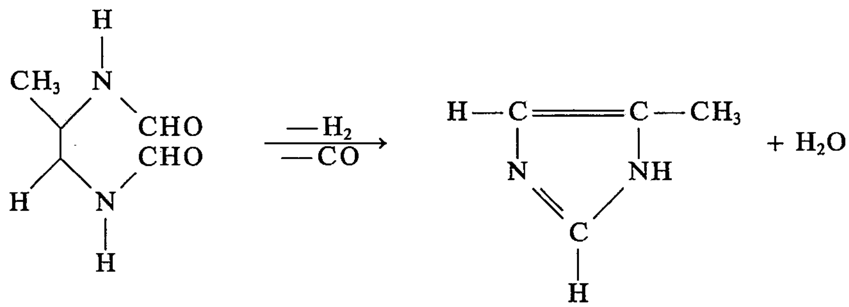


Im Vergleich zu den bekannten Verfahren liefert das Verfahren nach der Erfindung auf einfacherem und wirtschaftlicherem Wege Imidazole in besserer Ausbeute und Reinheit. Alle diese vorteilhaften Eigenschaften sind im Hinblick auf den Stand der Technik überraschend. So hätte man im Hinblick auf die DE-C-1 231 249 nicht vermuten können, daß gerade der erfindungsgemäße Katalysator, obwohl er nicht die 9 Komponenten des in der Patentschrift beschriebenen Katalysators enthält und sein Zinkoxid die erfindungsgemäße Struktur aufweist, höhere Ausbeuten an Endstoff liefert. Diese Ausbeuten waren um so überraschender, da die Umsetzung in Gegenwart von Kohlenmonoxid und Wasserstoff und somit im Gegensatz zu der Lehre der deutschen Patentschrift und der DE-A-2 729 017, die Stickstoff verwenden, durchgeführt wird. Es war weiterhin im Hinblick auf die Lehre der deutschen Offenlegungsschrift, daß Wasserstoff unzweckmäßig ist, bei der erfindungsgemäßen Verwendung von Wasserstoff zumindest eine geringere Ausbeute, wenn nicht eine Blockierung der Reaktion zu erwarten. Hinzu kommt, daß man bei Verwendung der erfindungsgemäßen Katalysatoren im Hinblick auf die Lehre der deutschen Offenlegungsschrift die Verwendung der freien Amine mit Ameisensäure und nicht die der N,N'-Diformylverbindungen als vorteilhafte Arbeitsweise betrachtet hätte. Vergleicht man die Lehren beider vorgenannter Veröffentlichungen zusammen mit dem erfindungsgemäßen Verfahren, so war es überraschend, daß die erfindungsgemäßen Ausgangsstoffe gerade mit den erfindungsgemäßen Katalysatoren und dazu in Gegenwart der erfindungsgemäßen Gase, insbesondere Wasserstoff, eine vergleichsweise höhere Ausbeute an Endstoff liefern.

Bevorzugte aliphatische Ausgangsstoffe II und dementsprechend bevorzugte Endstoffe I sind solche, in deren Formeln $R^1$ und $R^2$ gleich oder verschieden sein können und jeweils einen Alkylrest mit 1 bis 8 Kohlenstoffatomen bedeuten.

Als Ausgangsstoffe II kommen z. B. in Frage: Die N,N'-Diformylverbindungen von Äthylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin, 1,2-n-Decylendiamin, 1,2-n-Octadecylendiamin; 2,3-Butylendiamin, 2,3-Pentylendiamin, 2,3-Hexylendiamin, 2,3-Heptylendiamin, 2,3-Octylendiamin, 2,3-Nonylendiamin, 2,3-Decylendiamin, 3,4-Hexylendiamin, 3,4-Heptylendiamin, 3,4-Octylendiamin, 3,4-Nonylendiamin, 3,4-Decylendiamin, 4,5-Octylendiamin, 4,5-Nonylendiamin, 4,5-Decylendiamin, 5,6-Decylendiamin; durch die Benzyl-, Cyclohexyl- und/oder Phenylgruppe in 1-Stellung einfach oder in 1- und 2-Stellung gleichzeitig substituierten Äthylendiaminen; durch vorgenannte Alkylgruppen in 1-Stellung und durch die Benzyl-, Cyclohexyl- oder Phenylgruppe in 2-Stellung substituierten Äthylendiaminen.

Die Umsetzung wird bei einer Temperatur von 400 bis 500°C, zweckmäßig von 400 bis 460°C,

vorzugsweise von 425 bis 460°C, drucklos oder unter Druck, diskontinuierlich oder bevorzugt kontinuierlich durchgeführt. In der Regel dient das Reaktionsgemisch auch als Lösungsmedium.

Als Katalysator werden Zinkoxid allein oder ein Gemisch von Zinkoxid und Aluminiumoxid, zweckmäßig in einem Verhältnis von Zink zu Aluminium wie 1 bis 50, vorzugsweise 8 bis 10 Grammatom Zink je Grammatom Aluminium, und von 0,1 bis 1, vorzugsweise von 0,2 bis 0,4 Grammatom Zink je Mol Ausgangsstoff II verwendet. Als Aluminiumoxid kommen z. B. $\alpha$- und $\gamma$-Aluminiumoxid in Frage. Man kann auch Zinkverbindungen, die unter den Reaktionsbedingungen Zinkoxid ergeben, verwenden, z. B. ein mit Zinkchlorid oder Zinksulfat imprägniertes Aluminiumoxid. Ebenfalls kommen anstelle von Aluminiumoxid auch dieses Aluminiumoxid enthaltende Stoffgemische in Betracht, z. B. Aluminiumoxid enthaltende Aluminiumsilikat, Magnesiumaluminiumsilikathydrat, Dimagnesiumaluminiumsilikathydrat, Natriumaluminiumsilikat, Calciumaluminiumsilikat, Fullererde, Tone, Bleicherden wie Bentonit, Bauxit, Bimsstein, Andalusit, Kaolin, Allophane, Zeolithe, Mullit, Korund, $\gamma$-Tonerde, Hydrargillit, Böhmit.

Der Katalysator kann trägerfrei sein oder aich auf einem Träger, vorteilhaft in einer Menge von 1 bis 18 Gewichtsprozent Katalysator, bezogen auf den Träger, aufgebracht sein. Ebenfalls können vorgenannte Aluminiumverbindungen gleichzeitig in Gestalt des darin enthaltenen $Al_2O_3$ als Katalysatorkomponente und für das Zinkoxid als Träger dienen. Als Träger kommen zweckmäßig in Frage Kieselsäureverbindungen wie Silikate, z. B. Montmorillonit, Floridaerde, Quarz, Asbest; gefällte Kieselsäure, Kieselgel, Kieselgur; Titandioxid, Zirkondioxid, Zinndioxid, Aktivkohle; Erdalkalisulfate oder Erdalkaliphosphate, z. B. die Calcium- oder Bariumsalze; oder entsprechende Gemische vorgenannter Trägermaterialien. Die Herstellung der Trägerkatalysatoren wird nach den üblichen Verfahren, z. B. durch Auftragen der Zinkverbindung und gegebenenfalls der Aluminiumverbindung auf den Träger, Trocknen und Calcinieren, beispielsweise zwischen 400 und 1200°C in reduzierender, oxidierender oder inerter Atmosphäre, durchgeführt. Der Träger kann auch in seiner gewünschten geometrischen Form mit einer Lösung der Zinkverbindung allein bzw. der Zink- und Aluminiumverbindung, z. B. einer wäßrigen Lösung von Zinksulfat und gegebenenfalls Aluminiumsulfat, getränkt und getrocknet werden. Ebenfalls kann man das Trägermaterial mit der Zinkverbindung und gegebenenfalls der Aluminiumverbindung und Wasser verkneten, in die gewünschte Form bringen, trocknen und bei einer Temperatur von 400 bis 1200°C calcinieren.

Die Teilchengröße der Katalysatoren beträgt vorzugsweise von 0,005 bis 7, insbesondere 2 bis 4 Millimeter. Die Form kann beliebig, z. B. in Pillen-, Zylinder- oder Strangform, kugelförmig oder körnig, gewählt werden. Das Zinkoxid besitzt Porenvolumina von 0,05 bis 1 Milliliter je Gramm, vorzugsweise von 0,2 bis 0,4 Milliliter je Gramm, und spezifische Oberflächen von 1 bis 300 Quadratmeter je Gramm, vorzugsweise von 50 bis 100 Quadratmeter je Gramm, und zweckmäßig Schüttgewichte von 0,4 bis 2,1, vorzugsweise von 0,8 bis 1,4 Gramm je Milliliter. Man kann auch mit Katalysator überzogene Rohre oder netzartige Träger verwenden.

Vorzugsweise werden die Katalysatoren auf dem Träger in Splitt- oder Kugelform in der Wirbelschicht eingesetzt, wobei zweckmäßig Katalysatorteilchen mit Korngrößen von 0,005 bis 3 mm, insbesondere von 0,1 bis 1 mm, bevorzugt 0,2 bis 0,4 mm, verwendet werden. Die Schichthöhe des Katalysatorbettes im Wirbelzustand beträgt vorteilhaft 30 bis 2000 mm oder wird zweckmäßig so gewählt, daß sich Verweilzeiten der Ausgangsstoffe II in der Katalysatorschicht von 0,01 bis 20, vorzugsweise von 5 bis 10 Sekunden ergeben. Bezüglich der Herstellung der Katalysatoren wird auf Houben—Weyl, Methoden der Organischen Chemie, Band 4/2, Seiten 142 ff., und Ullmanns Encyklopädie der technischen Chemie, Band 9, Seiten 271 ff., verwiesen.

Die Umsetzung wird in Gegenwart von 1 bis 100, vorteilhaft 2 bis 50, insbesondere 3 bis 18 Mol Kohlenmonoxid und 1 bis 100, vorteilhaft 3 bis 50, insbesondere 6 bis 20 Mol Wasserstoff je Mol Ausgangsstoff II durchgeführt. Die Gemische können noch weitere gasförmige bzw. dampfförmige Komponenten enthalten. Bevorzugt wird das Abgas der erfindungsgemäßen Reaktion zurückgeführt (Kreisgas) und als Gasgemisch von $CO/H_2$ verwendet. Vorteilhaft ist ein Kreisgas von 30 bis 90, insbesondere 50 bis 75 Gewichtsprozent CO, von 3 bis 15, insbesondere 5 bis 7 Gewichtsprozent Wasserstoff, das im allgemeinen noch von 0 bis 50, insbesondere 10 bis 40 Gewichtsprozent $CO_2$, 0 bis 30, insbesondere von 0 bis 10 Gewichtsprozent Stickstoff, 0 bis 10, insbesondere 0,5 bis 5 Gewichtsprozent Ammoniak, 0 bis 15, insbesondere 0,5 bis 10 Gewichtsprozent dem Ausgangsstoff II zugrunde liegendes Diamin, 0 bis 12, insbesondere 0,1 bis 6 Gewichtsprozent Methan enthält.

Die Reaktion kann wie folgt durchgeführt werden: Der feste, verflüssigte oder zweckmäßig dampfförmige Ausgangsstoff II wird im Gemisch mit Wasserstoff und Kohlenmonoxid, zweckmäßig in Form des Abgases der Reaktion als Kreisgas, bei der Reaktionstemperatur über den Katalysator bzw. Katalysator auf dem Träger in einem Festbett geleitet. Das aus dem Reaktor dampfförmig austretende Reaktionsgemisch wird gegebenenfalls in einem Zyklon entstaubt und in einer gekühlten Vorlage kondensiert. Durch fraktionierte Destillation wird dann zweckmäßig der Endstoff abgetrennt. Der Endstoff kann auch durch Umkristallisation oder Umfällung aus geeigneten Lösungsmitteln, z. B. mit Toluol, Dimethylformamid, oder verdünnten Säuren, z. B. mit Ameisensäure, isoliert werden.

In einer bevorzugten Ausführungsform des Verfahrens wird der Ausgangsstoff II in einer Wirbelschicht bei der Reaktionstemperatur umgesetzt. Der Katalysator bzw. Katalysator auf Träger kann durch das Ausgangsgemisch oder das Gemisch von Wasserstoff und Kohlenmonoxid allein,

zweckmäßig in Gestalt des vorgenannten Kreisgases, als Wirbelschichtgas bei Normaldruck oder vermindertem oder erhöhtem Druck in einer Wirbelschicht gehalten werden. Entsprechend kann die Gesamtmenge oder eine Teilmenge an Ausgangsstoff II getrennt von dem Wirbelschichtgas in den Wirbelschichtreaktor eingeleitet werden. Das Diamin II wird zweckmäßig in einem beheizten Vorratsgefäß flüssig gehalten und in einen Verdampfer dosiert, der dem Wirbelschichtreaktor vorgeschaltet ist. Gleichzeitig leitet man vorteilhaft einen schwachen Kreisgasstrom, zweckmäßig von 5000 bis 50 000 Volumteilen des vorgenannten Kreisgases je Stunde, durch den Verdampfer. Der verdampfte Ausgangsstoff wird zusammen mit dem Kreisgasstrom durch das Katalysatorbett geleitet. Ebenfalls kann man aber auch den Ausgangsstoff II in fester Form, vorteilhaft in einer Korngröße zwischen 0,1 bis 3 mm, oder in flüssiger Form direkt in den Wirbelschichtreaktor eindosieren, wobei die Zudosierung des Ausgangsstoffs II getrennt von dem $CO/H_2$-Gemisch oder zusammen mit ihm erfolgen kann. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Band 1, Seiten 916 ff., verwiesen. Die Aufarbeitung des Reaktionsgemisches erfolgt in vorgenannter Weise.

In einer vorteilhaften Ausführungsform führt man nach der Reaktion das dampfförmige Reaktionsgemisch in eine Trennkolonne, zieht aus dem Sumpf den Endstoff ab und führt über den Kopf der Kolonne das restliche Gemisch in eine Vorlage, wo die kondensierbaren Anteile, im wesentlichen Wasser und das dem Ausgangsstoff I zugrunde liegende Diamin kondensiert werden. Ein Teil des Abgases, zweckmäßig ein Anteil, der die erfindungsgemäßen Mengen an CO und $H_2$ enthält, wird in eine Waschkolonne geführt, anschließend getrocknet und als Kreisgas zurückgeführt.

Die nach dem Verfahren der Erfindung herstellbaren Imidazole I sind wertvolle Ausgangsstoffe für die Herstellung von Farbstoffen, Pflanzenschutzmitteln, Textilhilfsmitteln, Katalysatoren für Polyurethane und Epoxidharze, oberflächenaktiven Mitteln und Pharmazeutika, z. B. den entsprechenden Nitroimidazolen. Die Imidazole I werden als Katalysatoren für Polymerisationsreaktionen und Aldolkondensationen verwendet. Bezüglich der Verwendung wird auf die vorgenannten Veröffentlichungen und Ullmanns Encyklopädie der technischen Chemie (3. Auflage), Band 8, Seite 499, und (4. Auflage) Band 13, Seiten 173 bis 175, verwiesen.

Die in den folgenden Beispielen aufgeführten Teile bedeuten Gewichtsteile. Die Gewichtsteile verhalten sich zu den Volumteilen wie Kilogramm zu Liter.

## Beispiel 1

a) 180 Teile 1,2-Diaminopropan werden unter Rühren und Kühlung mit 320 Teilen Ameisensäuremethylester bei 25 bis 30° C gemischt. Nach dem Abkühlen des Reaktionsgemisches auf 0° C wird das ausgefallene N,N-Diformyldiaminopropan (Fp. 65 bis 67° C) abgesaugt und getrocknet.

b) 90 Teile N,N'-Diformyl-diaminopropan werden pro Stunde aus einem Vorratsgefäß zusammen mit 550 000 Volumteilen pro Stunde Kreisgas durch einen auf 405° C erhitzten Wirbelreaktor geleitet. Das Kreisgas enthält 61,2 Gewichtsprozent CO (12,4 Mol je Mol Ausgangsstoff II), 6,3 Gewichtsprozent $H_2$ (17,7 Mol je Mol Ausgangsstoff II), 5,6 Gewichtsprozent $NH_3$, 5,0 Gewichtsprozent Methan und 21,9 Gewichtsprozent $CO_2$. Der Wirbelreaktor ist ein vertikales, elektrisch beheiztes Quarzrohr, das nach unten mit einer Quarzfritte abgeschlossen ist. Er ist mit 2500 Teilen eines Katalysators aus 90 Gewichtsprozent Zinkoxid (Porenvolumen 0,5 Milliliter je Gramm; spezifische Oberfläche 250 Quadratmeter je Gramm; Schüttgewicht 1,1 Gramm je Milliliter) und 10 Gewichtsprozent Aluminiumoxid (Korngröße 0,1 bis 0,3 mm) zur Hälfte gefüllt. Die Verweilzeit in der Katalysatorzone im Wirbelzustand beträgt 8,5 Sekunden. Die Höhe der Katalysatorzone beträgt 8,5 Sekunden. Die Höhe der Katalysatorzone beträgt im Wirbelzustand 800 mm. Die den Reaktor verlassenden Dämpfe werden kondensiert und fraktioniert destilliert. 550 000 Volumteile Abgas werden als Kreisgas in den Wirbelreaktor zurückgeführt. Man erhält stündlich 39,2 Teile (69,1% der Theorie, bezogen auf Ausgangsstoff II) 4-Methylimidazol vom Fp. 45° C. Der Umsatz beträgt 99,5 Prozent. Die Ausbeute blieb auch noch nach 300 Stunden Betrieb konstant.

## Beispiel 2

Stündlich werden 50 Teile N,N-Diformyldiaminopropan in einem Quarzverdampfer bei 300° C verdampft und die Dämpfe zusammen mit 310 000 Volumteilen pro Stunde Kreisgas durch einen auf 405° C erhitzten Festbettreaktor geleitet. Das Kreisgas enthält 73,4 Gewichtsprozent CO (14,4 Mol je Mol Ausgangsstoff II), 6,55 Gewichtsprozent $H_2$ (18,0 Mol je Mol Ausgangsstoff II), 2,3 Gewichtsprozent $NH_3$, 3,3 Gewichtsprozent Methan und 14,45 Gewichtsprozent $CO_2$. Der Reaktor enthält 5 mm lange und 2 mm dicke zylindrische Füllkörper, die aus 80 Teilen ZnO (Porenvolumen 0,3 Milliliter je Gramm; spezifische Oberfläche 180 Quadratmeter je Gramm; Schüttgewicht 1 Gramm je

Milliliter) und 20 Teilen γ-Aluminiumoxid bestehen. Nach der Aufarbeitung analog Beispiel 1 erhält man stündlich 18,1 Teile (57,3% der Theorie, bezogen auf eingesetzten Ausgangsstoff II) 4-Methylimidazol vom Schmelzpunkt Fp. 46°C. Der Umsatz beträgt 96,5 Prozent, bezogen auf das eingesetzte Diamid.

Beispiel 3

270 Teile N,N'-Diformyl-1,2-diamino-2-phenyl-äthylendiamin pro Stunde werden analog Beispiel 1 mit stündlich 750 000 Volumteilen Kreisgas bei 425°C umgesetzt. Das Kreisgas enthält 77,5 Gewichtsprozent CO (10,0 Mol je Ausgangsstoff II), 6,3 Gewichtsprozent $H_2$ (11,45 Mol je Mol Ausgangsstoff II), 2,25 Gewichtsprozent $NH_3$, 5,25 Gewichtsprozent $CH_4$ und 8,7 Gewichtsprozent $CO_2$. Man erhält 167,3 Teile 4(5)-Phenylimidazol vom Schmelzpunkt Fp. 128 bis 129°C (82,8% der Theorie, bezogen auf eingesetzten Ausgangsstoff II).

Beispiel 4

90 Teile pro Stunde N,N'-Diformyläthylendiamin werden analog Beispiel 1 zusammen mit stündlich 450 000 Volumteilen Kreisgas bei 420°C umgesetzt. Das Kreisgas enthält 49,9 Gewichtsprozent CO (7,8 Mol je Mol Ausgangsstoff II), 5,95 Gewichtsprozent $H_2$ (13 Mol je Mol Ausgangsstoff II), 3,0 Gewichtsprozent $NH_3$, 1,95 Gewichtsprozent Methan und 39,2 Gewichtsprozent $CO_2$. Man erhält stündlich 46,2 Teile Imidazol (87,6% Ausbeute, bezogen auf eingesetzten Ausgangsstoff II) vom Fp. 89°C.

Beispiel 5

100 Teile N,N'-Diformyl-2,3-diaminobutan und 550 000 Teile Kreisgas werden stündlich durch den auf 435°C erhitzten Wirbelschichtreaktor geleitet. Das Kreisgas enthält 76,3 Gewichtsprozent CO (15,9 Mol je Mol Ausgangsstoff II), 5,45 Gewichtsprozent $H_2$ (15,9 Mol je Mol Ausgangsstoff II), 2,05 Gewichtsprozent $NH_3$, 2,9 Gewichtsprozent Methan und 13,3 Gewichtsprozent $CO_2$. Man erhält analog Beispiel 1 stündlich 37,8 Teile (57,7% der Theorie, bezogen auf umgesetzten Ausgangsstoff II) 4,5-Dimethylimidazol vom Schmelzpunkt Fp. 112°C. Der Umsatz beträgt 98,3 Prozent, bezogen auf den eingesetzten Ausgangsstoff. Die Ausbeute bleibt auch nach 300 Stunden konstant.

**Patentanspruch**

Verfahren zur Herstellung von Imidazolen der Formel (I)

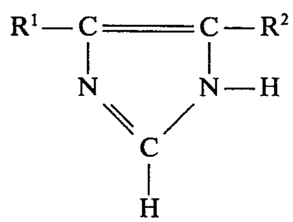

(I)

worin $R^1$ und $R^2$ gleich oder verschieden sein können und jeweils einen Alkylrest mit 1 bis 18 Kohlenstoffatomen, einen Cyclohexylrest, einen Aralkylrest oder Alkylarylrest mit 7 bis 12 Kohlenstoffatomen, einen Phenylrest oder ein Wasserstoffatom bedeuten, durch Umsetzung von N,N'-Diformyl-1,2-diaminen bei erhöhter Temperatur in Gegenwart von Zinkoxid und gegebenenfalls Aluminiumoxid, dadurch gekennzeichnet, daß man N,N'-Diformyl-1,2-diamine der Formel (II)

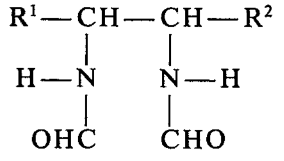

(II)

worin $R^1$ und $R^2$ die vorgenannte Bedeutung besitzen, bei einer Temperatur von 400 bis 500°C in Gegenwart von Zinkoxid mit einem Porenvolumen von 0,05 bis 1 Milliliter je Gramm und einer

spezifischen Oberfläche von 1 bis 300 Quadratmeter je Gramm oder einem Gemisch von diesem Zinkoxid und Aluminiumoxid oder dieses Aluminiumoxid enthaltenden Stoffgemischen als Katalysatoren mit oder ohne Träger und in Gegenwart eines Gemischs von Kohlenmonoxid und Wasserstoff mit einem Verhältnis von 1 bis 100 Mol Kohlenmonoxid und 1 bis 100 Mol Wasserstoff je Mol Ausgangsstoff II, gegebenenfalls im Gemisch mit $CO_2$, $NH_3$ und/oder $CH_4$, in der Gasphase umsetzt.

**Claim**

A process for the production of imidazoles of the formula (I)

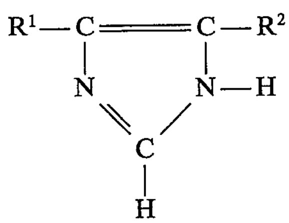

(I)

where $R^1$ and $R^2$ may be identical or different and each is alkyl of 1 to 18 carbon atoms, cyclohexyl, aralkyl or alkylaryl of 7 to 12 carbon atoms, phenyl or hydrogen, by reacting N,N'-diformyl-1,2-diamines at elevated temperature in the presence of zinc oxide and in the presence or absence of aluminium oxide, wherein N,N'-diformyl-1,2-diamines of the formula (II)

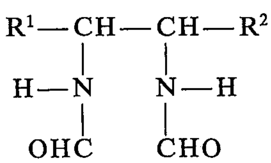

(II)

where $R^1$ and $R^2$ have the above meanings, are reacted in the gas phase at 400 to 500° C in the presence of zinc oxide having a pore volume of 0.05 to 1 milliliter per gram and a specific surface area of 1 to 300 $m^2$ per gram, or a mixture of zinc oxide and aluminium oxide or a mixture of compounds containing this aluminium oxide, as catalyst, which may be optionally supported, in the presence of a mixture of carbon monoxide and hydrogen, the molar ratio of carbon monoxide to hydrogen to starting material II being from 1 – 100 : 1 – 100 : 1, optionally in admixture with $CO_2$, $NH_3$ and/or $CH_4$.

**Revendication**

Procédé de préparation d'imidazoles de la formule (I)

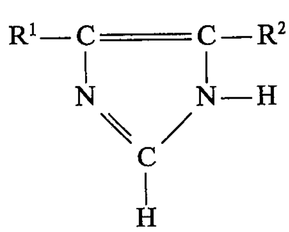

(I)

dans laquelle $R^1$ et $R^2$, qui peuvent être identiques ou différents, désignent chacun un radical alcoyle en $C_1$ à $C_{18}$, un groupe cyclohexyle, un groupe aralcoyle ou alcoylaryle en $C_7$ à $C_{12}$, un groupe phényle ou un atome d'hydrogène, par transformation de N,N'-diformyl-1,2-diamines à température accrue en présence d'oxyde de zinc et éventuellement d'oxyde d'aluminium, caractérisé en ce que l'on fait réagir des N,N'-diformyl-1,2-diamines de la formule (II)

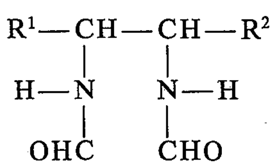

(II)

dans laquelle R[1] et R[2] possèdent la signification définie ci-dessus, en phase gazeuse, à une température comprise entre 400 et 500° C, en présence d'un oxyde de zinc avec un volume des pores de 0,05 à 1 ml par gramme et avec une surface spécifique de 1 à 300 m² par gramme, ou d'un mélange d'un tel oxyde de zinc et d'oxyde d'aluminium ou de mélanges de substances comprenant de l'oxyde d'aluminium comme catalyseurs sur support ou non et en présence d'un mélange d'oxyde de carbone et d'hydrogène avec des proportions de 1 à 100 moles d'oxyde de carbone et de 1 à 100 moles d'hydrogène pour 1 mole du composé de départ II, ce mélange pouvant le cas échéant encore renfermer du $CO_2$, du $NH_3$ et (ou) du $CH_4$.